# EUROPÄISCHE PATENTSCHRIFT

(11) **EP 0 714 881 B1**
(45) Veröffentlichungstag und Bekanntmachung des Hinweises auf die Patenterteilung: **31.03.1999**
(21) Anmeldenummer: 95118839.0
(22) Anmeldetag: 30.11.1995
(51) Int. Cl.: C07C 67/03, C07C 69/92

(54) **Verfahren zur Herstellung von Benzoesäure(4-hydroxyphenyl-ester)-Derivaten**
Process for the preparation of derivatives of (4-hydroxyphenyl-)esters of benzoic acid
Procédé de préparation de dérivés d'esters de 4-hydroxyphénol et d'acide benzoique

(30) Priorität: 01.12.1994 DE 4442831
(43) Veröffentlichungstag der Anmeldung: 05.06.1996
(73) Patentinhaber: Consortium für Elektrochemische Industrie GmbH, 81379 München (DE)
(72) Erfinder: Zahn, Ingo, Dr., D-80339 München (DE); Häberle, Norman, Dr., D-80689 Martinsried (DE); Weitzel, Peter, Dr., D-84571 Reischach (DE)
(74) Vertreter: Fritz, Helmut, Dr.

(56) Entgegenhaltungen:
- EP-A- 0 252 357
- PATENT ABSTRACTS OF JAPAN vol. 12 no. 128 (C-489) ,20.April 1988 & JP-A-62 249945 (FUJI PHOTO FILM CO LTD) 30.Oktober 1987,

## Beschreibung

Die Erfindung betrifft ein Verfahren zur Herstellung von Benzoesäure(4-hydroxyphenylester)-Derivaten.

Benzoesäure(4-hydroxyphenylester)-Derivate sind wichtige und bisher schlecht herstellbare organische Zwischenprodukte. Beispielsweise dienen Benzoesäure(4-hydroxyphenylester)-Derivate, welche in 1-Stellung des Benzoesäurerings einen Rest tragen, welcher eine Alkenyl- oder Alkinylgruppe aufweist, zur Herstellung flüssigkristalliner, siloxanhaltiger Seitenkettenpolymere.

Das Hauptproblem der Synthese von Benzoesäure(4-hydroxyphenylester)-Derivaten besteht darin, bifunktionelle Hydrochinonderivate nur auf einer Seite mit Benzoesäureverbindungen zu verknüpfen. Beim aus der US-A-5,211,877 bekannten Herstellungsverfahren wird Allyloxybenzoesäurechlorid mit einem sehr großen Überschuß an Hydrochinon zur Reaktion gebracht. Nachteil dieser Methode ist, daß der Überschuß an Hydrochinon nach der Reaktion umständlich wieder abgetrennt werden muß, was zu einer schlechten Raum-Zeit-Ausbeute an Allyloxybenzoesäure(4-hydroxyphenylester) führt.

In der JP-A-62 249945 ist die Herstellung von Hydrochinon-monoestern aliphatischer Carbonsäuren aus Hydrochinon-diestern aliphatischer Carbonsäuren mit Hilfe einer Base beschrieben.

In der EP-A-252 357 ist ein Verfahren zur Herstellung von (4-Hydroxyphenyl-)4-hydroxy-benzoat durch Veresterung von Hydrochinon und p-Hydroxybenzoesäure in Dispersion in Gegenwart eines Veresterungskatalysators beschrieben.

Die Aufgabe bestand nun darin, ein besseres Verfahren zur Herstellung von Benzoesäure(4-hydroxyphenylester)-Derivaten zu finden.

Die Erfindung betrifft ein Verfahren zur Herstellung von Benzoesäure(4-hydroxyphenylester)-Derivaten der allgemeinen Formel 1 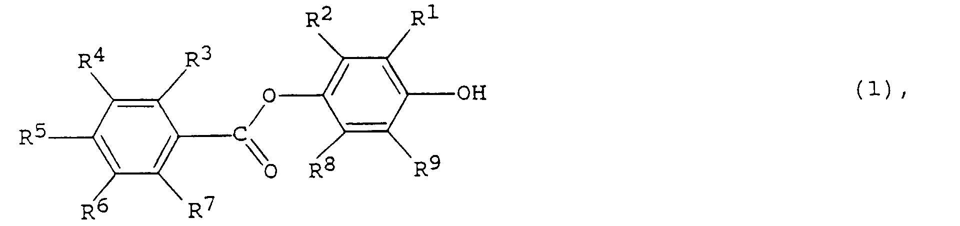 bei dem 1,4-Bis-(benzoyl)hydrochinonderivate der allgemeinen Formel 2 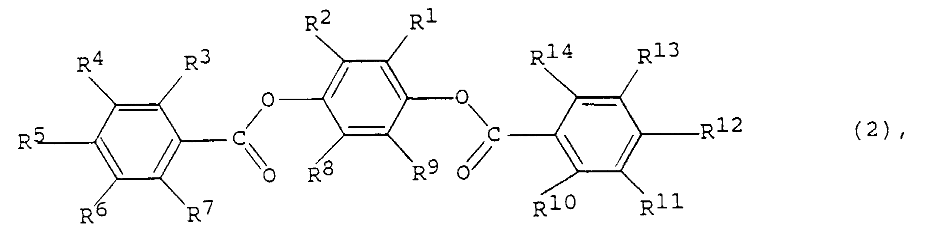 mit Alkalimetallalkanolaten umgesetzt werden,
wobei in den allgemeinen Formeln 1 und 2
**R**^{**1**}**, R**^{**2**}**, R**^{**3**}**, R**^{**4**}**, R**^{**6**}**, R**^{**7**}**, R**^{**8**}**, R**^{**9**}**, R**^{**10**}**, R**^{**11**}**, R**^{**13**} und **R**^{**14**} gleiche oder verschiedene Reste, die ausgewählt werden aus Wasserstoffatomen, Halogenatomen, Nitro- und Cyanogruppen, C₁- bis C₄-Alkoxy-, C₁- bis C₆-Alkyl-, Carboxy-(C₁- bis C₄-Alkyl)- und Tri-(C₁- bis C₄-Alkyl)-Siloxyresten und
**R**^{**5**} und **R**^{**12**} gleiche oder verschiedene Reste, die ausgewählt werden aus Wasserstoffatomen und C₁- bis C₂₀-Alkyl-, C₂- bis C₂₀-Alkenyl- und C₂- bis C₂₀-Alkinylresten, bei denen jeweils eine oder mehrere einander nicht benachbarte Methyleneinheiten durch Sauerstoffatome ersetzt sein können, bedeutet.

Bei diesem neuen allgemeinen Syntheseweg werden die 1,4-Bis-(benzoyl)hydrochinonderivate der allgemeinen Formel 2 in einer sauberen Reaktion mit sehr guter Ausbeute zu den entsprechenden Benzoesäure(4-hydroxyphenylester)-Derivaten der allgemeinen Formel 1 und zu den Benzoesäureester-Derivaten der allgemeinen Formel 3 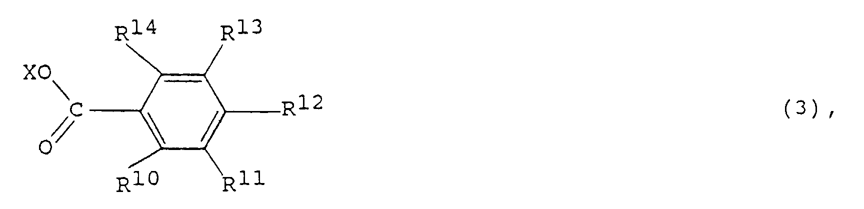 gespalten, wobei
**X** einen Alkylrest bedeutet und
**R**^{**10**}, **R**^{**11**}, **R**^{**12**}, **R**^{**13**} und **R**^{**14**} die bei den allgemeinen Formeln 1 und 2 angegebenen Bedeutungen aufweisen.

Die Benzoesäureester-Derivate der allgemeinen Formel 3 sind ebenfalls oft benötigte Zwischenprodukte in der organischen Chemie, beispielsweise für die Synthese anderer Mesogene, die in flüssigkristalline Silicone eingebaut werden können, und somit erwünschte Koppelprodukte.

Beipiele für die C₁- bis C₆-Alkylreste **R**^{**1**}, **R**^{**2**}, **R**^{**3**}, **R**^{**4**}, **R**^{**6**}, **R**^{**7**}, **R**^{**8**}, **R**^{**9**}, **R**^{**10**}, **R**^{**11**}, **R**^{**13**} und **R**^{**14**} sind der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentyl-cyclo-Pentylrest und Hexylreste, wie der n-Hexylrest und der cyclo-Hexylrest.

Beispiele für die C₁- bis C₄-Alkylreste, die als Bestandteil der C₁- bis C₄-Alkoxy-, Carboxy-(C₁- bis C₄-Alkyl)- und Tri-(C₁-bis C₄-Alkyl)-Siloxyreste **R**^{**1**}, **R**^{**2**}, **R**^{**3**}, **R**^{**4**}, **R**^{**6**}, **R**^{**7**}, **R**^{**8**}, **R**^{**9**}, **R**^{**10**}, **R**^{**11**}, **R**^{**13**} und **R**^{**14**} vorkommen, sind die vorstehend aufgeführten C₁- bis C₄-Alkylreste.

Als Halogenatome kommen bevorzugt Fluor, Chlor und Brom in Betracht.

Beispiele für C₁- bis C₂₀-Alkylreste **R**^{**5**} und **R**^{**12**} sind die vorstehend aufgeführten C₁- bis C₆-Alkylreste, Heptylreste, wie der n-Heptylrest, Oktylreste, wie der n-Octylrest und iso-Oktylreste, wie der 2,2,4-Trimethylpentylrest, Nonylreste, wie der n-Nonylrest, Decylreste, wie der n-Decylrest, Dodecylreste, wie der n-Dodecylrest, Oktadecylreste, wie der n-Oktadecylrest; Cycloalkylreste, wie Cyclopentyl-, Cyclohexyl-, Cycloheptylreste und Methylcyclohexylreste.

Beispiele für C₂- bis C₂₀-Alkenylreste **R**^{**5**} und **R**^{**12**} sind Vinyl-, Allyl-, Propenyl-(1)-, Butenylreste, wie der n-Butenyl-(1)-, n-Butenyl-(2)- und der Butadienyl-(1.3)-rest, Pentenylreste, wie der n-Pentenyl-(4)- und der Isoprenylrest, Hexenylreste, wie der n-Hexenyl-(5)-rest, Heptenylreste, Oktenylreste, Nonenylreste, Decenylreste, Dodecenylreste, Oktadecenylreste; Cycloalkenylreste, wie Cyclopentenyl-, Cyclohexenyl-, Cycloheptenylreste und Methylcyclohexenylreste.

Beispiele für C₂- bis C₂₀-Alkinylreste **R**^{**5**} und **R**^{**12**} sind Äthinyl-, Propinyl-(1)-, Propargyl-, Butinylreste, wie der Butinyl-(1)-, Butinyl-(2)- und der Butadiinylrest, Pentinylreste, wie der n-Pentinyl-(4)-rest, Hexinylreste, wie der n-Hexinyl-(5)- und der Hexatriinyl-(1.3.5)-rest, Heptinylreste, Oktinylreste, Noninylreste, Decinylreste, Dodecinylreste, Oktadecinylreste.

Beispiele für C₁- bis C₂₀-Alkyl-, C₂- bis C₂₀-Alkenyl- und C₂- bis C₂₀-Alkinylreste **R**^{**5**} und **R**^{**12**}, bei denen jeweils eine oder mehrere einander nicht benachbarte Methyleneinheiten durch Sauerstoffatome ersetzt sind, sind der n-Decyloxy-, Allyloxy-, n-Pentenyl-(4)-oxy- und n-Hexinyl-(5)-rest.

Bevorzugt als Reste **R**^{**5**} und **R**^{**12**} sind die C₁- bis C₁₅-Alkyloxy-, C₂- bis C₁₅-Alkenyloxy- und C₂- bis C₁₅-Alkinyloxyreste, insbesondere die linearen Reste. Bevorzugt sind die linearen Alkenyloxy- und Alkinyloxyreste, die nur eine Alkenyl- oder Alkinylgruppe in Endstellung aufweisen.

Insbesondere werden im Verfahren 1,4-Bis-(benzoyl)hydrochinonderivate der allgemeinen Formel 2 eingesetzt, bei denen **R**^{**5**} und **R**^{**12**} gleich sind.

Vorzugsweise werden als Alkalimetallalkanolate die Verbindungen der allgemeinen Formel 4

MOX (4),

eingesetzt, in der
**M** ein Lithium-, Natrium- oder Kaliumatom und
**X** einen C₁- bis C₆-Alkylrest bedeuten.

Beispiele für die C₁- bis C₆-Alkylreste X sind vorstehend aufgeführt.

Bei der Umsetzung wird vorzugsweise ein Verhältnis 1,0 bis 2,0, insbesondere 1,1 bis 1,7 Mol 1,4-Bis-(benzoyl)hydrochinonderivat der allgemeinen Formel 2 gegenüber dem Alkalimetallalkanolat gewählt.

Die Umsetzung wird vorzugsweise bei Temperaturen von 0 bis 120°C, insbesondere 30 bis 90°C durchgeführt. Vorzugsweise beträgt der Druck 0.05 - 1 MPa, insbesondere 0.09 - 0.2 MPa.

Die Spaltungsreaktion wird vorzugsweise in Lösemitteln durchgeführt. Falls Lösemittel verwendet werden, sind Lösemittel oder Lösemittelgemische, welche unter den Reaktionsbedingungen weitgehend inert sind, und insbesondere solche mit einem Siedepunkt bzw. Siedebereich von bis zu 120°C bei 0,1 MPa bevorzugt. Beispiele für solche Lösemittel sind Ether, wie Dioxan, Tetrahydrofuran, Diethylether, Methyl-tert.-butylether, Diethylenglycoldimethylether; chlorierte Kohlenwasserstoffe, wie Dichlormethan, Trichlormethan, Tetrachlormethan, 1,2-Dichlorethan, Trichlorethylen; Kohlenwasserstoffe, wie Pentan, n-Hexan, Hexan-Isomerengemische, Heptan, Octan, Waschbenzin, Petrolether, Benzol, Toluol, Xylole; Ketone, wie Aceton, Methylethylketon, Methylisobutylketon; Ester, wie Essigsäureethylester, Essigsäuremethylester, Essigsäure-n-butylester, Ameisensäureethylester; Schwefelkohlenstoff und Nitrobenzol, oder Gemische dieser Lösungsmittel. Die Bezeichnung Lösemittel bedeutet nicht, daß sich alle Reaktionskomponenten in diesem lösen müssen. Die Reaktion kann auch in einer Suspension oder Emulsion eines oder mehrerer Reaktionspartner durchgeführt werden. Die Reaktion kann auch in einem Lösemittelgemisch mit einer Mischungslücke durchgeführt werden, wobei in jeder der Mischphasen jeweils mindestens ein Reaktionspartner löslich ist.

Insbesondere werden als Lösemittel die aprotischen Lösemittel wie die cyclischen und acyclischen Ether eingesetzt.

### Beispiel

Eingesetzt werden:
222 g (0,516 mol) 1,4-Bis-(4-allyloxybenzoyl)hydrochinon (CAS-Nr. 85234-29-3)
29,55 g Natriummethylat
500 ml THF, 400 ml Petrolether (40-60°C)
200 ml verdünnte Schwefelsäure
Na₂SO₄ zur Trocknung

1,4-Bis-(4-allyloxybenzoyl)hydrochinon wird in THF suspendiert. Dann wird das Natriummethylat unter Rühren zugegeben. Anschließend wird noch zweieinhalb Stunden unter Rückfluß erhitzt und dann verdünnte Schwefelsäure zum Produkt zugegeben. Nach Beendigung des Rührens trennen sich zwei Phasen. Die untere, wäßrige Phase wird verworfen und die organische Phase mit Wasser nachgewaschen. Die Produktlösung wird über Na₂SO₄ getrocknet und dann eingedampft. Beim Abkühlen des Öles kristallisiert Monoallyloxybenzoesäurehydrochinon aus. Das Produkt wird nun mit 400 ml Petrolether aufgeschlämmt und abgesaugt. In der Petroletherphase ist der Allyloxybenzoesäuremethylester. Ausbeute: 90%d.Th.

## Patentansprüche

1. Verfahren zur Herstellung von Benzoesäure(4-hydroxyphenylester)-Derivaten der allgemeinen Formel 1 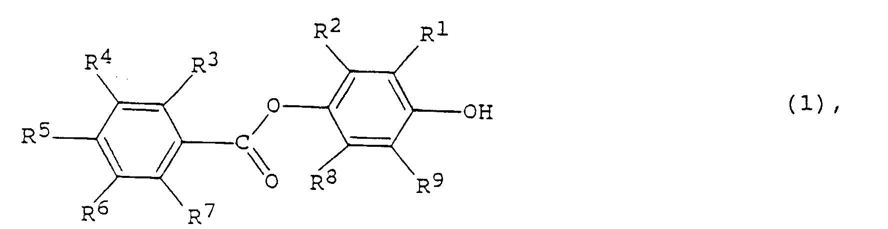
bei dem 1,4-Bis-(benzoyl)hydrochinonderivate der allgemeinen Formel 2 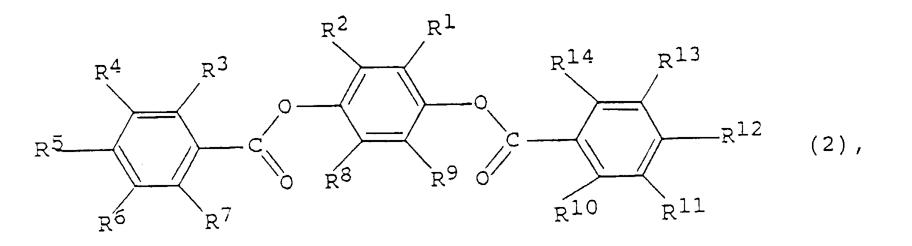 mit Alkalimetallalkanolaten umgesetzt werden,
wobei in den allgemeinen Formeln 1 und 2
**R**^{**1**}**, R**^{**2**}**, R**^{**3**}**, R**^{**4**}**, R**^{**6**}**, R**^{**7**}**, R**^{**8**}**, R**^{**9**}**, R**^{**10**}**, R**^{**11**}**, R**^{**13**} und **R**^{**14**} gleiche oder verschiedene Reste, die ausgewählt werden aus Wasserstoffatomen, Halogenatomen, Nitro- und Cyanogruppen, C₁- bis C₄-Alkoxy-, C₁- bis C₆-Alkyl-, Carboxy-(C₁- bis C₄-Alkyl)- und Tri-(C₁- bis C₄-Alkyl)Siloxyresten und
**R**^{**5**} und **R**^{**12**} gleiche oder verschiedene Reste, die ausgewählt werden aus Wasserstoffatomen und C₁- bis C₂₀-Alkyl-, C₂- bis C₂₀-Alkenyl- und C₂- bis C₂₀-Alkinylresten, bei denen jeweils eine oder mehrere einander nicht benachbarte Methyleneinheiten durch Sauerstoffatome ersetzt sein können, bedeuten.

2. Verfahren nach Anspruch 1, wobei
**R**^{**5**} und **R**^{**12**} C₁- bis C₁₅-Alkyloxy-, C₂- bis C₁₅-Alkenyloxy- oder C₂- bis C₁₅-Alkinyloxyreste bedeuten.

3. Verfahren nach Anspruch 1 oder 2, wobei 1,4-Bis(benzoyl)hydrochinonderivate der allgemeinen Formel 2 eingesetzt werden, bei denen **R**^{**5**} und **R**^{**12**} gleich sind.

4. Verfahren nach Anspruch 1 bis 3, in dem als Alkalimetallalkanolate die Verbindungen der allgemeinen Formel 4
MOX (4),
eingesetzt werden, wobei
**M** ein Lithium-, Natrium- oder Kaliumatom und
**X** einen C₁- bis C₆-Alkylrest bedeuten.

## Claims

1. Process for the preparation of 4-hydroxyphenyl benzoate derivatives of the general formula 1: 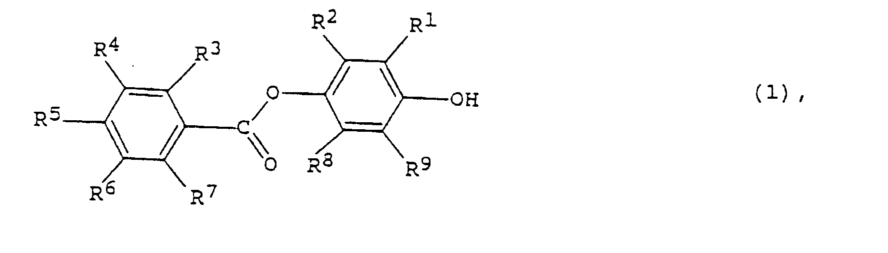 wherein 1,4-bis(benzoyl)hydroquinone derivatives of the general formula 2: 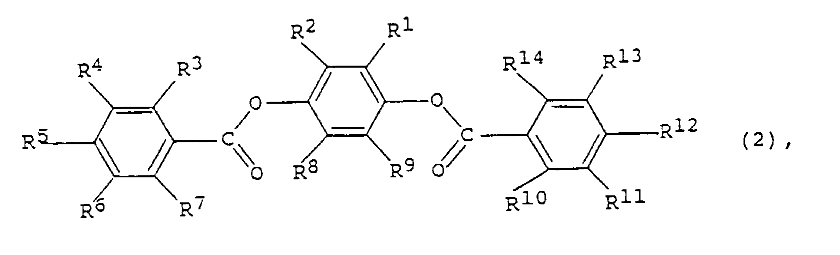 are reacted with alkali metal alkanolates,
in which general formulae 1 and 2:
R¹, R², R³, R⁴, R⁶, R⁷, R⁸, R⁹, R¹⁰, R¹¹, R¹³ and R¹⁴ are identical or different radicals selected from hydrogen atoms, halogen atoms, nitro and cyano groups and C₁- to C₄-alkoxy, C₁- to C₆-alkyl, carboxy(C₁- to C₄-alkyl) and tri(C₁- to C₄-alkyl)siloxy radicals and
R⁵ and R¹² are identical or different radicals selected from hydrogen atoms and C₁- to C₂₀-alkyl, C₂- to C₂₀-alkenyl and C₂- to C₂₀-alkynyl radicals, it being possible in each case for one or more non-adjacent methylene units to be replaced with oxygen atoms.

2. Process according to Claim 1 wherein R⁵ and R¹² are C₁- to C₁₅-alkoxy, C₂- to C₁₅-alkenyloxy or C₂- to C₁₅-alkynyloxy radicals.

3. Process according to Claim 1 or 2 wherein 1,4-bis-(benzoyl)hydroquinone derivatives of the general formula 2 are used in which R⁵ and R¹² are identical.

4. Process according to Claims 1 to 3 wherein the alkali metal alkanolates used are the compounds of the general formula 4:
MOX (4),
wherein
M is a lithium, sodium or potassium atom and
X is a C₁- to C₆-alkyl radical.

## Revendications

1. Procédé de préparation de dérivés de benzoate de 4-hydroxyphényle de formule générale 1 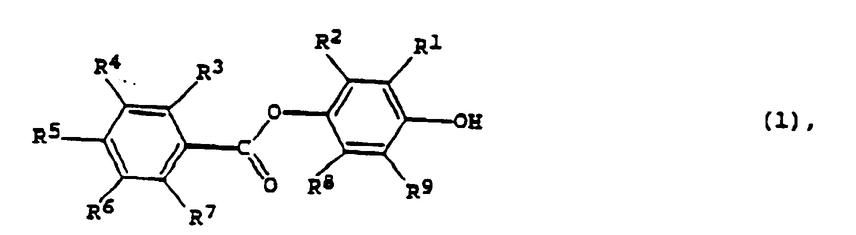 dans lequel on fait réagir des dérivés de 1,4-bis(benzoyl)hydroquinone de formule générale 2 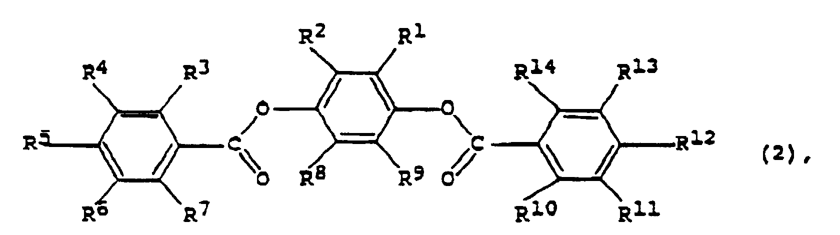 avec des alcanolates de métaux alcalins,
où, dans les formules générales 1 et 2,
R¹, R², R³, R⁴, R⁶, R⁷, R⁸, R⁹, R¹⁰, R¹¹, R¹³ et R¹⁴ représentent des radicaux identiques ou différents, qui sont choisis parmi des atomes d'hydrogène, des atomes d'halogène, des groupes nitro et cyano, des radicaux alcoxy en C₁ à C₄, alkyle en C₁ à C₆, carboxy(C₁-C₄-alkyle) et tri(C₁-C₄-alkyle)siloxy et
R⁵ et R¹² représentent des radicaux identiques ou différents, qui sont choisis parmi des atomes d'hydrogène et des radicaux alkyle en C₁ à C₂₀, alcényle en C₂ à C₂₀ et alcynyle en C₂ à C₂₀, pour lesquels, dans chaque cas, un ou plusieurs motifs méthylène non adjacents les uns aux autres peuvent être remplacés par des atomes d'oxygène.

2. Procédé selon la revendication 1, dans lequel R⁵ et R¹² représentent des radicaux alkyloxy en C₁ à C₁₅, alcényloxy en C₂ à C₁₅ ou alcynyloxy en C₂ à C₁₅.

3. Procédé selon la revendication 1 ou 2, dans lequel on utilise des dérivés de 1,4-bis(benzoyl)hydroquinone de formule générale 2, pour lesquels R⁵ et R¹² sont identiques.

4. Procédé selon les revendications 1 à 3, dans lequel on utilise, en tant qu'alcanolates de métaux alcalins, les composés de formule générale 4
MOX (4),
dans laquelle
M représente un atome de lithium, de sodium ou de potassium et
X représente un radical alkyle en C₁ à C₆.
